# EUROPEAN PATENT APPLICATION

(11) **EP 4 763 101 A2**
(43) Date of publication of application: **24.06.2026**
(21) Application number: 26163053.7
(22) Date of filing: 24.10.2023
(51) Int. Cl.: A61B 17/11

(54) **MEDICAL TISSUE WRAP**

(30) Priority: 26.10.2022 WO PCT/EP2022/079994
(62) Divisional of application: 23798159.2
(71) Applicant: TISSIUM S.A., 75012 Paris (FR)
(72) Inventor: MALAFOSSE, Marie, 75010 Paris (FR); LAMAZOUADE, Julien, 33460 Arsac (FR); COLLIN, Estelle, 94140 Alfortville (FR); PEREIRA, Maria, 1200-366 Lisbon (PT)
(74) Representative: Graf von Stosch Patentanwaltsgesellschaft mbH

(57) **Abstract**

The invention relates to the field of medical tissue wraps for internal anatomical structures as well as a use and a method of treating a nerve lesion using such medical tissue wraps. In particular, the invention relates to medical tissue wraps for neural tissue supporting the repair of peripheral nerve lesions by facilitating secure application of the medical tissue wrap. Accordingly, a medical tissue wrap (10) for an internal anatomical structure of a mammal is suggested, wherein the medical tissue wrap (10) is formed by a coiled wall (12) having a circumferential inner end portion (20) and a circumferential outer end portion (22) extending around and overlapping the circumferential inner end portion (20). The wall (12) thereby defines a longitudinal through-hole (16) for accommodating a portion of the anatomical structure. According to one aspect of the invention, the circumferential outer end portion (22) is radially spaced-apart from the circumferential inner end portion (20) by a discontinuous spacing in the circumferential direction. According to a further aspect, the tissue wrap comprises a structural outer surface modification at opposing longitudinal end portions of its wall.

## Description

### Technical field

The invention relates to medical tissue wraps for internal anatomical structures of a mammal as well as a use and a method of treating a nerve lesion using such medical tissue wraps. In particular, the invention relates to medical tissue wraps for neural tissue supporting the repair of peripheral nerve lesions.

### Technological Background

Upon injury of a person, tissue damage may occur to internal anatomical structures, such as muscular tissue, tendons, blood vessels, or neural tissue, which typically extend in a longitudinal direction and may comprise an essentially cylindrical or tube-like shape. Such tissue damage may result in impaired motor skills and/or in a partial sensory loss, requiring treatment to facilitate, at least in part, recovery of the affected tissue und functionality.

The impaired motor skills and/or partial sensory loss may be particularly pronounced in case of damage to neural tissue, which may involve one or more nerve lesions or traumata of the peripheral nerve system. Such injuries involving nerve damage particularly occur in the upper extremities, such as a hand or finger of a person, such that a person may experience a loss e.g. in tactile or haptic feedback and/or may have difficulties in controlling fine motor skills in the injured region, if the nerve lesion is not treated properly.

Current treatments of nerve lesions include coaptation of the nerve ends by various suturing techniques so as to provide a connection between the respective nerve ends in an essentially tensionless manner. In case a more severe defect is present, wherein the nerve ends are not directly adjacent to each other, a reconstruction may be required to overcome a corresponding gap. A reconstruction may be provided e.g. by an autologous or allogenic nerve graft. Alternatively, a reconstruction may be performed by providing a tubular structure so as to provide a nerve guide. Such tubular structure may be provided e.g. by autologous or allogenic venous structures or by artificially manufactured nerve conduits made of a biocompatible material.

The use of a tubular structure may furthermore facilitate the repair of the lesion irrespective of the presence of a gap, e.g., by providing further mechanical support and structural stability, providing a tensionless repair, providing guidance to axonal growth limiting the risk of neuroma, reducing an inflammatory response to the site of the lesion, and/or limiting the extent of fibrous tissue development.

Such tubular structure may also be provided in the form of a medical tissue wrap, e.g. a sheet material, which may be coiled or rolled around the damaged internal tissue. The medical tissue wrap may e.g. be applied to a peripheral nerve that has been prepared by guiding the medical tissue wrap in an at least partially uncoiled state around the designated portion of the nerve and by allowing or ensuring recoiling of the tissue wrap after proper positioning. Contrary to a conduit, the medical tissue wrap may hence advantageously also be applied to damaged anatomical structures that are not separated by a gap, but comprise, at least partially, a continuous longitudinal extension. Closure of the medical tissue wrap is then typically ensured by a plurality of sutures at the outer circumference.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" e.g., a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The term "about" in relation to a numerical value x means x ± 10%.

In numerical values with decimal places, comma (",") or point (".") are used interchangeably herein, i.e. throughout the present specification and claims. Accordingly, numerical values with decimal places may be expressed with either comma (",") or point ("."). For example, the exemplary value of "0.5" may also be expressed as "0,5", which likewise applies to other values with decimal places. In particular, a comma (",") in a numerical value indicates decimal places, but is not used as "thousands separator".

### Summary of the invention

Starting from the known prior art there is a need to further facilitate the repair of damaged internal anatomical structures, in particular of nerve or tendon lesions.

According to the invention it has been recognized that proper closure and securing of a medical tissue wrap being applied to an internal anatomical structure, e.g. a damaged peripheral nerve or tendon, are frequently difficult to achieve. Such closure and securing are required to avoid the accommodated damaged tissue from inadvertently protruding out of the medical tissue wrap and, vice versa, to avoid undesirable tissue growth, in particular of scar tissue or fibrous tissue, into the medical tissue wrap. In other words, undesirable communication between the damaged tissue and the surrounding tissue is preferably avoided by means of proper closure and securing of the medical tissue wrap.

Closure and securing of the medical tissue wrap may be improved by providing excess material, i.e. by increasing the number of layers of the medical tissue wrap being wrapped around the damaged anatomical structure (as described in US 9,061,464) or by integrating interlocking portions to flexible body that facilitate its wrapping and attachment around tissue (as described in EP 3662940). However, the present inventors have found that such additional wrapping or interlocking system may be cumbersome to perform *in situ* or even anatomically impossible. Furthermore, the addition of the number of layers may render it more difficult to provide the medical tissue wrap in an at least partially unrolled state, wherein a gap is provided that is sufficient to insert and/or accommodate the damaged anatomical structure. The additional material may furthermore result in an increased radial dimensioning of the medical tissue wrap and/or may alter the overall characteristics of the medical tissue wrap, e.g. in terms of structural stability, resilience, optics, and/or biochemical properties. The provision of additional layers may furthermore affect the inner diameter of the medical tissue wrap, such that over dimensioning may be required to avoid compression of the damaged anatomical structure.

As described above, closure and securing of the medical tissue wrap may also be ensured by means of a plurality of sutures. However, this complicates the surgical procedure, since, on the one hand, perforation of the delicate and damaged anatomical structure is generally to be avoided as it generates tension that has been associated with poorer tissue regeneration, on the other hand, the structural stability or integrity of the medical tissue wrap may be reduced. The provision of sutures furthermore increases the time required to perform the surgical procedure.

In addition, the provision of additional layers and/or the provision of sutures renders it more difficult to perform the surgical procedure in a reproducible manner.

The reproducibility of the surgical procedure may also be diminished by the provision of sutures at longitudinal ends of the medical tissue wrap in an attempt to secure the medical tissue wrap to the accommodated anatomical structure. Such securing means may furthermore be inadequate for the anatomical structure to be accommodated by the medical tissue wrap or may complicate the surgical procedure and/or handling of the delicate tissue. Alternatives using a medical adhesive have been found difficult to apply in a reproducible manner and/or to maintain a secured state of the medical tissue wrap to the damaged internal anatomical structure.

It is hence an object of the present invention to further facilitate the repair of damaged internal anatomical structures, e.g. nerve or tendon lesions, and, in particular, to ensure proper closure and/or securing of a medical tissue wrap without impairing the insertion of the internal anatomical structure into the medical tissue wrap.

Said object is achieved by the independent claims. Preferred embodiments are depicted in the dependent claims, the description, and the Figures.

Accordingly, in a first aspect, a medical tissue wrap for an internal anatomical structure of a mammal is provided, wherein the medical tissue wrap is formed by a coiled wall having a circumferential inner end portion and a circumferential outer end portion extending around and overlapping the circumferential inner end portion. The wall thereby defines a longitudinal through-hole for accommodating at least one portion of the anatomical structure. According to the invention, the circumferential outer end portion is radially spaced-apart from the circumferential inner end portion by a discontinuous spacing in the circumferential direction.

In general, the circumferential outer end portion of the wall and the circumferential inner end portion of the wall correspond essentially to the mutually overlapping inner and outer portions of the wall. The circumferential extension of the circumferential outer end portion hence essentially corresponds to the circumferential extension of the circumferential inner end portion of the wall, such that said outer end portion (fully) overlaps the inner end portion. Thereby, a circumferential gap of the coiled wall is avoided, ensuring that at least one internal anatomical structure being accommodated within the through-hole is retained within the medical tissue wrap and is essentially fully covered and/or surrounded along its (full) circumference. Accordingly, the medical tissue wrap may provide an improved supporting, protecting and/or suspending function for the anatomical structure being accommodated therein.

The circumferential outer end portion is radially spaced-apart (entirely) from the circumferential inner end portion. In other words, the circumferential outer end portion is not in any direct contact with the circumferential inner end portion due to the radial spacing between the inner and outer end portions. Due to the radial spacing for the entire overlapping portion in the coiled state of the medical tissue wrap, uncoiling or unrolling of the medical tissue wrap, at least in part, may be facilitated compared with medical tissue wrap configurations, wherein the one or more (outer) layers are in direct contact with a respectively overlapping and adjacent (inner) layer. The overlapping portions are hence spaced apart and do not contact each other in the coiled state of the wall. Thereby, a gap sufficient to insert the internal anatomical structure into the through-hole may be provided. Furthermore, such radial spacing may facilitate the optional application of a medical adhesive, e.g. to provide a bonding between the outer end portion and the inner end portion once the medical tissue wrap has been appropriately applied and positioned.

According to first embodiment, the radial spacing is discontinuous. As used herein, a "discontinuous" spacing means that the radial distance between the circumferential outer end portion and the circumferential inner end portion varies along the circumferential extension of the radial spacing (i.e., the circumferential extension of the overlap). Accordingly, the distance between the circumferential outer end portion and the circumferential inner end portion is usually smaller in one portion of the radial spacing as compared to another portion of the (same) radial spacing. Accordingly, a portion having a reduced radial spacing (but not providing a contacting surface between the circumferential inner and outer end portions) is usually present in the overlapping portion. The discontinuous spacing furthermore provides that at least an overlapping portion having a reduced radial spacing is present, which facilitates the closure of the through-hole towards the surrounding tissue environment. Thereby, the risk of external tissue growth into the through-hole may be effectively reduced while at the same time the retaining of the anatomical structure within the through-hole is improved. Furthermore, the discontinuity may define a barrier for an optionally applied medical adhesive at the outer surface and/or interface between the outer end portion and inner end portion, which may be dimensioned based on a viscosity of the medical adhesive to be used and a required minimum radial spacing to facilitate uncoiling or unrolling of the medical tissue wrap.

In other words, the discontinuous spacing ensures that an improved closure of the medical tissue wrap may be provided while still enabling the temporary provision of a circumferential gap between the outer end portion and inner end portion to facilitate the accommodation of damaged internal anatomical structures, e.g. a lesioned nerve or tendon.

Preferably, the medical tissue wrap is configured to accommodate neural tissue, preferably a lesioned peripheral nerve. The medical tissue wrap is configured to accommodate a proximal end and distal end of a lesioned single peripheral nerve, e.g. with a predefined gap therebetween due to trauma or in an essentially gapless manner.

Hence, the through-hole may be adapted and dimensioned to accommodate a corresponding diameter of a nerve or nerve end. For example, the diameter of the through-hole (which is understood to be the diameter of the inner portion) may be from 1 mm to 12 mm, depending on the diameter of the nerve (end). To accommodate smaller nerves, the diameter of the through-hole may e.g. be from 1 or 1,5 mm to 6,5 mm (e.g.1 mm, 1,5mm, 2mm, 2,5mm, 3mm, 3,5mm, 4mm, 4,5mm, 5mm, 5,5mm, 6mm or 6,5mm). Such dimensions have been found to be particularly advantageous to provide a required support for the nerve (ends) and/or nerve growth or repair. Depending on the desired therapeutic effect of the medical tissue wrap and the nerve, the extension of the wall and through-hole is preferably from 5 mm to 25 mm, more preferably from 7 mm to 22 mm (e.g. 7mm, 7,5mm, 8mm, 8,5mm, 9mm, 9,5mm, 10mm, 10,5mm, 11mm, 11,5mm, 12mm, 12,5mm, 13mm, 13,5mm, 14mm, 14,5mm, 15mm, 15,5mm, 16mm, 16,5mm, 17mm, 17,5mm, 18mm, 18,5mm, 19mm, 19,5mm, 20mm, 20,5mm, 21mm, 21,5mm or 22mm), in the longitudinal direction. Such dimensions may furthermore be particularly advantageous to bridge nerve defects or lesions. The desirable or predefined dimensions may thereby provide a continuous guiding structure for the internal anatomical structure that is particularly advantageous to direct and/or facilitate neurogenesis.

While the above preferred dimensions relate to (peripheral) nerve applications, it is to be understood that the medical tissue wrap may also be applied to provide a supporting structure for tendons, blood vessels (veins and/or arteries), or small ligaments. Accordingly, such supporting structure may be adapted to the corresponding dimensions, tissue properties and/or level of support to be provided. For example, the diameter of the through-hole may be chosen so as to ensure direct contact with the internal anatomical structure, e.g. for very delicate tissue structures, or to provide a predefined radial spacing between the wall and the internal anatomical structure, e.g. to provide a buffer upon impact on adjacent tissue. By the same token, the wall thickness may be chosen based on the required structural support and resilience of the wall in view of the internal anatomical structure to be treated.

To facilitate the application of the medical tissue wrap, the wall preferably defines a through-hole having a tubular shape with a continuous inner diameter. Such configuration also corresponds to the typically cylindrical and/or tube-like extension of the preferred internal anatomical structure to be accommodated within the through-hole. Furthermore, this provides that a predefined and/or more homogeneous structural stability of the medical tissue wrap may be achieved. The wall defining the through-hole may, optionally, comprise grooves, internal structures and/or holes at its inner surface. Such (inner) wall modifications may also improve the mechanical properties of the medical tissue wrap and/or facilitate repair of the internal anatomical structure retained within the through-hole, e.g. by promoting nerve growth to a lesioned peripheral nerve. However, in some embodiments, the inner surface of the wall defining the through-hole may be smooth, i.e. may not comprise grooves, internal structures and/or holes.

The tubular shape may provide that essentially continuous outer dimensions may be provided, which may be advantageous for implanting the medical tissue wrap with regard to the surrounding tissue. Furthermore, the tubular or cylindrical shape may provide sufficient structural stability and/or may prevent sharp bends or kinking during tissue movement, i.e. compression or extension. The tubular shape may accordingly also provide that a homogeneous structure is provided, which reacts in a predefined manner when forces act upon the wall, e.g. upon impact.

The tubular structure may furthermore essentially correspond to the shape of the through-hole, such that a continuous guiding structure for tissue repair or support (protection), e.g. neurogenesis or nerve growth, is provided, without providing an undesirable biasing. Thereby, achieving that the dimensioning of the through-hole may be kept to a desirable and/or required minimum.

The wall, in particular its inner surface, may also contain a drug, e.g. by means of coating or integrated in the material of the wall, which may be released over time and may e.g. facilitate tissue repair. In some embodiments, the wall may be covered/coated with a composition, for example a gel, containing such as drug (the through-hole may thus be filled with the composition at least partially or fully). For example, the drug may be a biologically active agent, e.g. antiinflammatory, immunosuppressant, neurotrophic factor and neuroprotective agent. Examples of such biologically active agents are cytokines, nerve growth factor, hyaluronic acid, tacrolimus, cyclosporin A, melatonin, vitamin B12, methylprednisolone, riluzole, taxol, cetuximab, 4-aminopyridine (4-AP), Tesamorelin; a preferred example is tacrolimus.

The circumferential outer end portion is preferably formed by a single wall layer overlapping the circumferential inner end portion. Said circumferential inner end portion is preferably formed by a single wall layer. Thereby, application of the medical tissue wrap may be significantly facilitated compared with configurations having a plurality of adjacent layers overlapping each other in the radial direction. For example, in case of a gapless internal anatomical structure to be treated, the single wall layers may facilitate uncoiling and recoiling of the medical tissue wrap to accommodate the internal anatomical structure.

Although a variety of coiled configurations of the wall may be provided, the wall is preferably only coiled in a (single or double) circumferential direction, in particular around one or two longitudinal axes extending through (along) the through-hole. Thereby, helical extensions of the wall are avoided. In other words, the wall preferably coils around a single or two longitudinal axes (corresponding to the circumferential outer end portion of the wall and the circumferential inner end portion of the wall, respectively), yet does not advance in the direction of the longitudinal axis. Accordingly, an essentially continuous and/or gapless circumferential cover of the internal anatomical structure may be provided. This may not only provide an improved support and/or protection of the accommodated tissue, but may also facilitate an uncoiling and/or recoiling upon application of the medical tissue wrap.

Preferably, the circumferential inner end portion and the circumferential outer end portion are arranged at circumferentially opposing end portions of the wall. Accordingly, the wall does not extend circumferentially beyond the circumferential inner end portion and the circumferential outer end portion, neither in the coiled state nor in a partially uncoiled state. In the coiled state, the wall preferably defines an essentially cylindrical shape of the medical tissue wrap, wherein the cross-section of the through-hole is preferably essentially circular. By providing the outer and inner end portions at circumferentially opposing end portions of the wall, the radial dimensioning of the medical tissue wrap may be reduced in the coiled state.

As described above, the discontinuous spacing advantageously comprises at least a portion defining a reduced radial spacing, wherein the radial spacing is to be understood as being a radial distance between the radially directly adjacent surfaces of the circumferential inner end portion and the circumferential outer end portion.

Preferably, the discontinuous spacing comprises a reduced radial spacing formed by a curvature of the circumferential inner end portion and/or a curvature of the circumferential outer end portion. The reduced radial spacing may be particularly formed as a gradually reduced spacing and/or may extend from an overlapping portion having a continuous radial spacing.

A curvature may e.g. be provided at the circumferential outer end portion, wherein an overlapping portion defines a gradually reduced radial spacing, i.e. in a circumferential direction from the circumferential inner end portion to the circumferential outer end portion or from the circumferential outer end portion to the circumferential inner end portion. By the same token, a curvature may be provided at the circumferential inner end portion, wherein an overlapping portion defines a gradually reduced radial spacing, i.e. in a circumferential direction from the circumferential outer end portion to the circumferential inner end portion or from the circumferential inner end portion to the circumferential outer end portion.

Such curvatures may be provided separately, i.e. either a (gradual) reduction in a circumferential direction from the circumferential inner end portion to the circumferential outer end portion or from the circumferential outer end portion to the circumferential inner end portion. However, such curvatures may also be provided simultaneously (in the same wrap), wherein a continuous radial spacing and/or a local maximum radial spacing is provided between said curvatures. In this regard, it may also be provided that a reduced radial spacing is formed by two curvatures of the circumferential outer end portion, which define a gradually reduced radial spacing in opposing circumferential directions and surrounding the circumferential inner end portion having an essentially continuous pitch or radius. Similarly, it may also be provided that a reduced radial spacing is formed by two curvatures of the circumferential inner end portion, which define a gradually reduced radial spacing in opposing circumferential directions surrounded by the circumferential outer end portion having an essentially continuous pitch or radius.

In some embodiments, the radial spacing at a circumferential end face of the circumferential inner end portion is less than the radial spacing at a circumferential end face of the circumferential outer end portion. Thereby, the radial spacing may be (gradually) reduced towards the circumferential end face of the circumferential inner end portion. A maximum radial spacing may thus be provided at the circumferential end face of the circumferential outer end portion; and/or a minimum radial spacing may be provided at the circumferential end face of the circumferential inner end portion. For example, the radial spacing at a circumferential end face of the circumferential inner end portion may be about 80-99%, preferably 85-95%, more preferably about 90% of the radial spacing at a circumferential end face of the circumferential outer end portion. Moreover, the minimum radial spacing may be about 80-99%, preferably 85-95%, more preferably about 90% of the maximum radial spacing.

Preferably, the radial spacing at a circumferential end face of the circumferential outer end portion is less than the radial spacing at a circumferential end face of the circumferential inner end portion. Thereby, the radial spacing may be (gradually) reduced towards the circumferential end face of the circumferential outer end portion. A maximum radial spacing may thus be provided at the circumferential end face of the circumferential inner end portion; and/or a minimum radial spacing may be provided at the circumferential end face of the circumferential outer end portion. For example, the radial spacing at a circumferential end face of the circumferential outer end portion may be about 80-99%, preferably 85-95%, more preferably about 90% of the radial spacing at a circumferential end face of the circumferential inner end portion. Moreover, the minimum radial spacing may be about 80-99%, preferably 85-95%, more preferably about 90% of the maximum radial spacing.

The provision of the reduced radial spacing at an end face, wherein the radial spacing is preferably gradually reduced towards the respective end face, provides that a closure of the through-hole may be significantly improved.

In some embodiments, the radial spacing at a circumferential end face of the circumferential inner end portion and at a circumferential end face of the circumferential outer end portion is less than the radial spacing at an overlapping portion or point between the circumferential end face of the circumferential inner end portion and the circumferential end face of the circumferential outer end portion (thereby providing a maximum radial spacing, which is neither at the circumferential end face of the circumferential inner end portion nor at the circumferential end face of the circumferential outer end portion).

In particular, it has been found that a reduced radial spacing at both the circumferential end face of the circumferential inner end portion and at the circumferential end face of the circumferential outer end portion may provide a further improvement of the closure towards the through-hole by forming a double barrier, ensuring that the internal anatomical structure is securely retained within the through-hole and the risk of external tissue growth into the through-hole is reduced to the largest extent. It is to be understood that such configuration may be provided by corresponding curvatures as described above, wherein an overlapping portion between the end faces and/or between such curvatures may define a continuous radial spacing or a local maximum radial spacing.

Depending on the required closure and the dimensioning of the medical tissue wrap, the reduced radial spacing may also be provided only at the circumferential end face of the circumferential outer end portion, which may accordingly provide a sufficient outer closure of the overlapping portion while defining a more rounded outer surface of the medical tissue wrap.

By the same token, the reduced radial spacing may also be provided only at the circumferential end face of the circumferential inner end portion, which may accordingly provide a sufficient inner closure of the overlapping portion while facilitating opening or uncoiling of the medical tissue wrap due to the larger radial spacing at the circumferential end face of the circumferential outer end portion. Such configuration may also facilitate the optional application of a medical adhesive, e.g. by forming a longitudinal groove defined by the decreased radial spacing adjacent to the respective end face. While the reduced radial spacing itself may be sufficient to maintain the closure of the medical tissue wrap and to obviate the provision of one or more sutures at the wall, the provision of such groove for the application of a medical adhesive may be advantageous to provide an improved retaining force and to provide a material bonding between the circumferential inner end portion and the circumferential outer end portion with improved reproducibility.

In some embodiments, the radial spacing at a circumferential end face defining the reduced radial spacing may be from 50 percent to 99 percent of a maximum radial spacing of the discontinuous spacing, preferably from 60 percent to 99 percent, more preferably from 80 to 98 percent, even more preferably from 85 to 95 percent, still more preferably from 87 to 92 percent, e.g. about 90 percent. The reduced spacing may e.g. depend on the inner diameter defined by the through-hole and/or by the outer diameter of the wall and/or depend on the circumferential extension of the circumferential outer end portion.

The radial spacing at a circumferential end face defining the reduced radial spacing is preferably from 50 µm to 300 µm. In particular, said radial spacing may be from 80 µm to 200 µm. By the same token, the maximum radial spacing of the discontinuous spacing is preferably from 100 µm to 350 µm and may be particularly be from 150 µm to 250 µm. For example, the maximum radial spacing may be about 200 µm while the reduced (or minimal) radial spacing may be from 100 µm to 190 µm. Such dimensioning may provide an optimized closure of the medical tissue wrap in the coiled state of the wall while the radial spacing also facilitates uncoiling of the wall to accommodate the internal anatomical structure upon application of the medical tissue wrap. Furthermore, such dimensioning has been found to be particularly advantageous for closure of medical tissue wraps with smaller inner diameters of the through-hole from 1 mm to 6,5 mm or to 5 mm (e.g. 1mm, 1,5mm, 2mm, 2,5mm, 3mm, 3,5mm, 4mm, 4,5mm, 5mm, 5,5mm, 6mm or 6,5mm), e.g. suitable for small diameter peripheral nerves.

In order to ensure that no circumferential spacing is provided between the circumferential outer end portion and the circumferential inner end portion in the coiled state of the wall the overlapping portion may be accordingly dimensioned. Accordingly, the circumferential extension of the circumferential outer end portion may correspond to from 5 degrees to about 120 degrees, preferably from 10 degrees to 100 degrees of the outer circumference of the tissue wrap, more preferably from 20 degrees to 90 degrees.

The wall may hence form less than 1,5 coils, i.e. the overlapping portion provided by the circumferential outer end portion defines less than half a coil along the circumference. While the preferred degrees indicate the portion of the circumferential outer end portion in reference to the circumference of the medical tissue wrap as a whole in the coiled state (and forming 360 degrees, i.e. in reference to a single full coil without overlaps), said degrees may also be defined as a corresponding percentage. Accordingly, the circumferential extension of the circumferential outer end portion may correspond to from about 2,8 percent to about 28 percent, preferably from about 5,6 percent to about 25 percent of the outer circumference of the medical tissue wrap.

More preferably, the circumferential extension of the circumferential outer end portion may be from 20 degrees to 60 degrees of the outer circumference of the tissue wrap, even more preferably from 30 degrees to 45 degrees. In accordance with the above, said angular extensions may correspond to from about 5,6 percent to about 17 percent or from about 8,3 percent to about 13 percent of the outer circumference of the medical tissue wrap.

It has been found that the overlapping portion provided by the circumferential outer end portion may be advantageously chosen based on the inner diameter of the through-hole, wherein the circumferential extension is preferably smaller than 45 degrees for inner diameters up to about 3 mm, e.g. for smaller peripheral nerves of the digits or hand of a patient to be treated, and wherein the circumferential extension of more than 20 degrees and up to 45 degrees may be chosen for inner diameters of about 4 mm and more, e.g. for larger peripheral nerves such as those of the wrist or arm of a patient to be treated. The circumferential extension may be particularly more than 25 degrees, e.g. about 30 degrees or from about 30 degrees to about 45 degrees of the outer circumference of the medical tissue wrap.

Together with the discontinuous spacing, i.e. including a portion with a reduced radial spacing, such circumferential extensions have been found to provide improved closure of the medical tissue wrap and its through-hole while still facilitating the opening or uncoiling of the medical tissue wrap for the application at the target tissue. For circumferential extensions larger than 45 degrees such uncoiling may not be facilitated to the same extent, but such larger extensions may be chosen based e.g. on the internal anatomical structure to be accommodated within the through-hole and/or the required mechanical and/or closing properties of the medical tissue wrap.

The radial spacing between the circumferential inner end portion and the circumferential outer end portion may also be gradually reduced in opposing circumferential directions starting from a maximum radial spacing of the discontinuous spacing and with equal angular extension. As described above, such gradual reduction may be provided by a corresponding curvature having a pitch or radius differing from an adjacent wall portion, wherein said curvatures at outer and/or inner end portions may have the same angular extension, e.g. each of about 30 degrees of the outer circumference of the medical tissue wrap, so that the circumferential outer end portion forms an overall angular extension of about 60 degrees.

While the medical tissue wrap may be applied to an internal anatomical structure being separated by a gap, e.g. due to trauma or dissection, wherein opposing ends of said anatomical structure may be inserted into the through-hole of the medical tissue wrap via corresponding openings at opposing longitudinal ends of the through-hole, such application may be cumbersome and/or undesirable for small diameter anatomical structures and/or in terms of reproducibility.

Accordingly, the wall preferably is at least partially uncoilable and recoilable in a circumferential direction. Preferably, the wall may be fully uncoilable, such that the wall may define a sheet-like structure extending only in a longitudinal direction. By ensuring that the wall is uncoilable, the wall may be wrapped around the internal anatomical structure to be treated, e.g. a lesioned peripheral nerve or tendon and may be recoiled after proper positioning of the wall.

The wall may be at least partially formed of a resilient or elastic material, preferably of a three-dimensional printed material. Such resilience may not only be advantageous to provide a desired support and/or level of flexibility adapted to surrounding tissue movements, but may also facilitate an uncoiling and recoiling of the wall to apply the medical tissue wrap around the internal anatomical structure to be treated. Such resilience may e.g. be provided by means of three-dimensional printing, wherein local stresses in the material may provide corresponding biasing forces.

Depending on the structural support provided by the medical tissue wrap to the internal anatomical structure, the wall thickness may be accordingly chosen. Preferably, the wall comprises a thickness from 50 µm to 400 µm, preferably from 150 µm to 250 µm. Such thickness has been found to provide sufficient support for a variety of applications while at the same time ensuring a level of transparency, such that the accommodation of the internal anatomical structure within the through-hole may be monitored or examined once the wall is in the coiled state. Depending on the material used for said wall, said thickness may also be adapted to a required biodegradation rate of the medical tissue wrap. Furthermore, such thickness may facilitate uncoiling of the wall and may improve flexibility for handling of the medical tissue wrap. The preferred thickness range may e.g. be advantageous for uncoiling of the wall. A wall thickness of about 200 µm has been found to be particularly advantageous for the above aspects.

While the wall comprises a continuous portion having said preferred thickness range, local increases in wall thickness may be provided to provide or support further functionalities of the medical tissue wrap.

In particular, a reduced radial spacing may be provided by means of a curvature of the circumferential inner end portion. In some embodiments, a reduced radial spacing may be provided by a (gradual) increase in the wall thickness of the circumferential inner end portion and/or in the adjacent section of the non-overlapping portion of the wall. While such increase in the wall thickness may generally be applied to the medical tissue wrap, it may be particularly advantageous at such portions of the medical tissue wrap having structural outer surface modifications, in particular retention surfaces, as described herein below. Accordingly, such increase in the wall thickness is preferably at a longitudinal end portion having structural outer surface modifications, in particular retention surfaces, as described herein below.

Accordingly, in circumferential direction, the wall of the circumferential inner end portion may be thinner as compared to the wall of the outer end portion and of (the majority of) the non-overlapping circumferential portion of the wall. In particular if the medical tissue wrap comprises structural outer surface modifications, such as retention surfaces, and, thus, an increased wall thickness at a longitudinal end portion as described herein below, the thickness of the wall of the circumferential inner end portion at such a longitudinal end portion may be about the same as the thickness of the wall at the central portion (where the wall thickness is preferably essentially the same in the entire circumferential direction).

In some embodiments, the thickness of the wall of the circumferential inner end portion may be reduced by up to 75%, preferably from 30% to 65%, more preferably from 40% to 60%, such as about 50%, as compared to the thickness of the wall of the circumferential outer end portion and/or (the majority of) the non-overlapping circumferential portion of the wall.

However, also the non-overlapping circumferential portion of the wall may contain a section having a reduced wall thickness, which is directly adjacent to the circumferential inner end portion having a reduced wall thickness. Thereby, a smooth transition may be provided between (i) the (essentially constant) wall thickness of the outer end portion and the (majority of the) non-overlapping circumferential portion of the wall; and (ii) the inner end portion having a reduced wall thickness in comparison to (i). Accordingly, the thickness of the wall may be gradually reduced (starting) in the section of the non-overlapping circumferential portion of the wall towards the inner end portion, reaching a minimum thickness of the wall (in circumferential direction) at the (circumferential end face of the) inner end portion. Thereby, sharp edges are preferably avoided, such that a homogeneous and stepless outer surface is preferably provided that is free of sharp edges.

The section of the non-overlapping circumferential portion of the wall, which has a reduced wall thickness, may cover an angle (circumferential offset) in circumferential direction of 1° to 90°, preferably 2° to 45°, more preferably 3° to 30° and even more preferably 5° to 20°.

Due to the inner end portion and, optionally, additionally the adjacent section 35 of the non-overlapping circumferential portion of the wall 12, having a reduced wall thickness, manufacturing may be facilitated and the circumferential outer end portion 22 may be better aligned with the adjacent outer circumference of the wall. Furthermore, this may provide that the handling of the medical tissue wrap 10, in particular the accessibility of the circumferential outer end portion 22 is facilitated, such that an uncoiling of the wall 12 and the medical tissue wrap 10 may be supported, both by providing a visual cue and by providing a small gripping recess.

In order to ensure that a relative movement between the internal anatomical structure and the medical tissue wrap is reduced or even avoided under normal physiological behavior, the application of a medical adhesive to secure the internal anatomical structure to the medical tissue wrap may be advantageous.

In a second aspect, the present invention also provides a medical tissue wrap for an internal anatomical structure of a mammal, the medical tissue wrap being formed by a coiled wall having a circumferential inner end portion and a circumferential outer end portion extending around and overlapping the circumferential inner end portion thereby defining a longitudinal through-hole for accommodating a portion of the internal anatomical structure, wherein the circumferential outer end portion is radially spaced-apart from the circumferential inner end portion and wherein the wall comprises a structural outer surface modification at opposing longitudinal end portions of the wall. The detailed description of this wrap essentially corresponds to the above detailed description of the medical tissue wrap according to the first aspect, with the only difference that the radial spacing in the wrap according to the second aspect is not necessarily discontinuous. Additionally, the medical tissue wrap according to the second aspect comprises a structural outer surface modification at least at one opposing longitudinal end portions of its wall. Otherwise the above (and following) description of the first aspect corresponds to the second aspect.

Accordingly, a medical tissue wrap according to the present invention may have either a discontinuous spacing as described above, or continuous spacing combined with a structural outer surface modification at least at one opposing longitudinal end portions of its wall. Preferably, however, the medical tissue wrap comprises both features, i.e. a discontinuous spacing as described above and a structural outer surface modification at least at one (preferably both) opposing longitudinal end portions of its wall. Accordingly, the wall of the medical tissue wrap according to the first aspect preferably comprises a structural outer surface modification at least at one opposing (preferably both) longitudinal end portions of the wall. Likewise, the wrap according to the second aspect preferably comprises a typically discontinuous spacing as described in detail above.

With regard to the wall comprising a structural outer surface modification at opposing longitudinal end portions of the wall, the wall preferably defines a longitudinally extending central portion arranged between and adjacent to the opposing longitudinal end portions, wherein only the longitudinal end portions (or at least one) comprise the structural outer surface modification. At least one of the longitudinal end portions, most preferably both, comprises an increased surface roughness formed by the structural outer surface modification and compared with the surface roughness of the outer surface of the central portion.

The structural outer surface modification and/or the increased surface roughness has the advantage that a bonding strength of an optionally applicable medical adhesive at said modified surface may be improved, such that a securing and/or anchoring of the medical adhesive to the respective surface may be strengthened. For example, the modification may provide an increased surface area and/or a form-fitting for the applied medical adhesive and/or may improve the adherence due to a modified surface tension. Thereby, the medical adhesive, which is applied to the modified surface and the adjacent tissue of the internal anatomical structure protruding out of the through-hole, may ensure that the medical tissue wrap is held in place and/or maintains secured in the appropriate position relative to the internal anatomical structure that is treated.

The structural outer surface modifications may be formed on a thickened wall portion of the respective longitudinal end portion compared with the wall thickness of the central portion. For example, the thickness of the thickened wall portion may be from 1,1 to 5,0 fold the thickness of the adjacent central portion, preferably from 1,5 to 2,5 fold. The thickness of the thickened wall portion may be from 100 µm to 600 µm, preferably from 300 µm to 500 µm. As described above, the wall portion that is not locally modified may comprise a thickness from 50 µm to 400 µm, preferably from 150 µm to 250 µm.

Alternatively, it may also be provided that a continuous and essentially homogeneous thickness of the wall is provided, wherein the structural outer surface modifications are embedded in the wall. In such case, the overall wall thickness may be adapted to the structural outer surface modifications to ensure structural stability at the portion that has been modified, e.g. having a minimum wall thickness of about 50 µm, preferably (at least) from 50 µm to 100 µm.

The thickened wall portion may be circumferentially offset from the circumferential inner end portion, wherein the offset preferably comprises a gradual increase of the wall thickness from the inner end portion to the thickened wall portion (at the non-overlapping circumferential wall portion). The circumferential offset may have a circumferential extension from 5 degrees to 25 degrees of the outer circumference of the medical tissue wrap. Preferably said circumferential extension of the offset is from 10 degrees to 20 degrees, more preferably about 15 degrees of the outer circumference of the medical tissue wrap. Accordingly, the circumferential inner end portion is free from a structural outer surface modification and may have a corresponding (reduced) wall thickness. The offset has the advantage that the alignment of the circumferential outer end portion to the circumferential inner end portion may be facilitated and that handling of the circumferential outer end portion may be facilitated, e.g. for the purpose of uncoiling the wall. The gradual transitioning between the thickened wall portion and the wall portion of the circumferential inner end portion ensures that sharp edges or steps may be omitted.

The structural outer surface modifications are preferably formed as one or more retention surfaces, e.g. at least partially external structures with particular geometries or surface irregularities, which are configured for securing a medical adhesive to the respective longitudinal end portion. Thereby, the medical adhesive that has been applied to the respective longitudinal end portion region and/or an adjacent tissue portion of the internal anatomical structure may remain in situ without any significant movement, even after curing of the medical adhesive. In other words, by means of the retention surfaces, the medical adhesive may be held in place, preferably in a form fitting manner with the one or more retention surfaces. The retention surfaces may provide that a contact surface with the medical adhesive is increased. Furthermore, it may provide that a predefined surface roughness is provided, thereby improving the efficacy of the medical adhesive to bond with the respective longitudinal end portion. Accordingly, a loosening or slipping of the medical adhesive may be effectively prevented by means of the shape and/or surface of the retention surface.

The retention surfaces of a respective longitudinal end portion may be formed as a plurality of (e.g., ellipsoid or circular) holes, that may be arranged in at least one row in a circumferential direction of the wall. The holes may hence be positioned in a linear fashion along the circumference of the wall. The number of rows preferably ranges from 1 to 10 rows. Each row preferably comprises 2 to 20 holes. The holes may advantageously be equally spaced apart in the circumferential direction of the elongate body. Preferably, the holes are arranged in 2 to 4 rows and/or each row comprises 4 to 8 holes. Thereby, the holes of adjacent rows are preferably arranged in a staggered formation. For example, two rows may be provided at a respective end region, wherein each row may comprise e.g. six holes.

The number of holes and rows as well as their arrangement have been found to be advantageous in order to provide and maintain the required structural stability, while simultaneously providing an improved hold for securing a medical adhesive to the medical tissue wrap. Furthermore, such embodiments may exhibit a rotational symmetry, which may facilitate the correct placement of the medical tissue wrap at the target tissue site. In certain embodiments, the size or diameter of the holes may be from 50 µm to 750 µm and, for further improved structural stability, preferably ranges from 150 µm to 600 µm and may particularly be about 500 µm.

Each hole may be formed as a cut-out, a recess, or a radial through-hole in the wall. Thereby, an improved securing of the medical adhesive may be provided. In particular, a radial through-hole may be further advantageous. Such a radial through-hole may facilitate that the medical adhesive surrounds the wall at the respective longitudinal end portion, in particular both, at the exterior and in the interior of the medical tissue wrap, which may depend on the chosen dimensions and/or, in particular, on the diameter of the holes. By the same token, this may also provide that the medical adhesive is brought into contact with the internal anatomical structure accommodated in the through-hole. The holes may hence have a radial height or depth corresponding to a strength or thickness of the wall, but may also have a reduced height. Preferably, the holes have a minimum height or depth of about 50 µm. The holes may have a height or depth of 50 µm to 500 or 600 µm, more preferably of 150 µm to 300 or 400 µm.

In some embodiments, the one or more retention surfaces of a respective longitudinal end portion may be preferably formed as at least one groove extending in a helical direction along a longitudinal axis defined by the wall. The helical shape may define a thread extending along the outer surface of the respective longitudinal end portion.

Although the one or more grooves may define sharp edges, the at least one groove preferably comprises rounded edges. The rounded edges are to be understood such that at the interface of the groove with the top outer surface of the respective longitudinal end portion and/or at an opposing bottom surface of the groove with the side wall (of the groove) no straight (angled) edges or steps are present. Instead a gradual transition may be provided at corner surfaces of the groove. By means of the rounded edges, stresses within the material of the wall may be reduced, resulting in a reduced occurrence of rupture or breakage. Accordingly, the provision of rounded edges may also reduce the required thickness of the wall at least at the longitudinal end portion to support the groove.

Alternatively, or (preferably) additionally to the round edges, the at least one groove may define at least one undercut. The provision of an undercut may improve the anchoring of the medical adhesive to the respective longitudinal end portion. The undercut may be formed such that a bottom portion of the groove is at least partially covered by the outer surface of the wall. That is, the outer wall surface may at least partially extend over the groove in a longitudinal direction. Preferably, such extension forms an angle (between the bottom and the sidewall of the groove) of from 45 degrees to 90 degrees, preferably from 60 to 85 degrees, more preferably from 70 degrees to 80 degrees, even more preferably about 75 degrees or 90 degrees. Thereby, a form-fitting or positive locking between an applied medical adhesive and the respective longitudinal end portion may be improved.

In order to reduce the radial extension of the elongate body and the nerve wrap as a whole, the at least one groove may define an outermost edge of the respective longitudinal end portion in a longitudinal direction of the wall. The groove may hence end at the longitudinal end surfaces of the wall and extend along the circumference at said end surface such that the radial extension may be reduced at said end. Thereby, the overall dimensioning of the medical tissue wrap may be reduced. Alternatively, the groove may also end at a longitudinal offset to said longitudinal end surface, which may be advantageous for further improving structural stability.

The depth or (top) longitudinal extension of the groove may be essentially constant along the circumference and throughout its longitudinal extension. Preferably, the groove may have a minimum depth of about 50 µm. The groove may have a depth of 50 µm to 500 µm, more preferably of 150 µm to 300 µm. Preferably, the groove may have a minimum (top) longitudinal extension of about 20 µm. The groove may have a thickness (width) of 10 µm to 100 µm, more preferably of 15 µm to 50 µm. According to the invention, the (top) longitudinal extension between positive and negative grooves may be equal or different.

The extension and/or the angle of the at least one groove may vary. Preferably, the at least one groove extends from 0,5 to 10 revolutions around a longitudinal axis defined by the wall, thereby providing a helical structure (at the longitudinal end portion(s)). Preferably, the groove extends for more than one revolution around the longitudinal axis defined by the wall. In particular, the number of revolutions may be from 2 to 6, preferably 3 to 5 or 4.

In some embodiments, the longitudinal end portion (or each of two longitudinal end portions) may comprise a single groove. In other embodiments, the respective longitudinal end portion(s) may also comprise at least two grooves. In particular if two or more grooves are provided in the same longitudinal end portion, each groove may extend from 0,5 to 5 revolutions around a longitudinal axis. The at least two grooves may be extending in parallel, for example forming two or more parallel (non-intersecting) helices. Thereby, arc length, curvature and torsion of the parallel grooves/helices are preferably the same, such that they only differ in their location (on the longitudinal end portion). Preferably, the distances between two or more parallel grooves/helices are regular (i.e. about the same).

In some embodiments, at least two grooves may extend in opposing circumferential directions and intersect each other. Both embodiments may also be combined, i.e. with two or more grooves extending in parallel and two or more additional grooves extending in opposing circumferential directions, optionally also in parallel (such that the parallel intersecting grooves provide a "checkered" or "pineapple" pattern). The number of grooves in each circumferential direction may be from 1 to 10 and preferably is from 6 to 8 or 7, depending on the longitudinal extension of the respective end portion and the angle of the grooves. The number of grooves is furthermore preferably equal for each circumferential direction. Moreover, at a single longitudinal end portion (or at each end portion), the grooves/helices preferably differ only in their direction (and location on the end portion), but preferably not in other helix parameters. In other words, arc length, curvature and torsion may be essentially the same for all grooves/helices at a certain longitudinal end portion (except for the direction of intersecting helices). By providing a plurality of grooves (e.g., two or more grooves) in each circumferential direction a diamond-shaped or rhombic-shaped pattern is provided at the outer surface of the respective longitudinal end portion, which may resemble a pineapple surface. Thereby, a plurality of retention surfaces with a large number of edges may be provided, which may be advantageous for securing the medical adhesive to the respective longitudinal end portion.

In some embodiments, the one or more retention surfaces of a respective end portion may be formed as one or more circumferential ribs (elongated protrusions) extending from an outer surface of the wall. The extension of the one or more ribs may be linear (e.g. parallel) to the circumferential direction or comprise an offset to the circumferential direction in a longitudinal direction of the wall (e.g. diagonal). Preferably, in case of a plurality of ribs, e.g. from 3 to 6, at the respective end portion, the ribs may be equally spaced apart from each other and do not intersect, i.e. are preferably arranged in parallel to each other.

The one or more retention surfaces may generally be provided on a thickened wall portion at the respective longitudinal end portion that is adapted to the implementation of the desired retention surface and the structural requirements at the longitudinal end portion. Examples of preferred thicknesses or thickness ranges have been described in the above. The thickened wall portion may be brought flush with the outer surface of the central portion by appropriate rounding or provision of fillets.

By the same token, the one or more holes and, in particular, grooves may also be provided as positive features at the outer surface of the wall having an otherwise essentially continuous thickness. The wall may hence comprise positive protrusions or build-ups at the respective longitudinal end portion defining the respective groove or hole while the wall portion between the opposing longitudinal end portions does not comprise such positive features. In such case, the outer surface of the wall hence preferably defines the inner radius of the respective groove or hole.

In order to increase the efficacy of a medical adhesive and, preferably, a form-fitting or interlocking of the medical adhesive with the medical tissue wrap, the retention surfaces may furthermore be at least partially comprised on a portion of the wall being directly adjacent to the respective longitudinal end portion. For example, one or more grooves may extend over the wall portion directly adjacent to the longitudinal end portion while exhibiting no increased wall thickness, e.g. originate from or terminate into a central or intermediate portion of the wall. Such extension of the retention surfaces may provide a gradual transition of the retention surfaces while improving the load distribution on the wall (and medical tissue wrap as a whole).

It may be provided that only one respective end portion of the medical tissue wrap is provided with the one or more retention surfaces. Preferably, both of the longitudinally opposing end portions, i.e. two opposing end portions, are provided with one or more retention surfaces. The longitudinal end portions (of the same medical tissue wrap) may have different types of retention surfaces, i.e. one longitudinal end portion may comprise a groove while another end portion comprises a plurality of holes. Preferably, however, the longitudinal end portions have equally formed retention surfaces.

The retention surfaces of opposing end portions may be mirrored about a transverse mid plane of the wall (or the medical tissue wrap), for example, such that they are aligned in opposing directions. For example, each of the longitudinally opposing end portions (of the same medical tissue wrap) may comprise one or more grooves/helices as described above, wherein the grooves or helices of the different longitudinal end portions may essentially correspond to each other (e.g., having the same number and/or pattern), but wherein the different longitudinal end portions differ from each other in that the grooves/helices are formed in a mirrored arrangement about a transverse mid plane of the wall (or medical tissue wrap as a whole), such that they are aligned in opposing directions. For example, on one longitudinal end portion, the groove may extend in clockwise direction, while on the other longitudinal end portion it extends in counter-clockwise direction. Alternatively, however, the retention surfaces of the different longitudinal end portions of the same medical tissue wrap may also be oriented or arranged in the same direction, e.g. have a groove or thread in the same (clockwise or counter-clockwise) direction.

According to special embodiment, the outermost edge of the respective longitudinal end portion may be offset in a longitudinal direction of the wall, with a decreased thickness of the wall in this offset portion (this can increase amount of medical adhesive applied on the edge of the medical tissue wrap). According to this special case, the retention surfaces when present may be comprised on a portion of the wall between the said offset of the longitudinal end portion and the central portion.

The wall of the medical tissue wraps as described herein may be formed of a biocompatible material, an inert material, a bioimplantable material, and/or a biodegradable material. The material may be chosen so as to provide a predefined structural stability while essentially avoiding or at least reducing the inflammatory response of a patient to be treated. For example, a biocompatible material may be chosen, which is gradually degrading over time after implantation yet which may initially provide sufficient structural support to adequately repair e.g. a nerve lesion during movement of the tissue.

For improved therapeutic result, the particular material may furthermore be advantageously chosen in order to facilitate or support repair, e.g. nerve growth, for example, by comprising or otherwise incorporating or including a corresponding coating, e.g. with a biologically active agent and/or one or more neurotrophic factors. Other examples of such biologically active surface functionalities include, but are not limited to, e.g. anti-inflammatories, immunosuppressants, neurotrophic factors and neuroprotective agents. Examples of such biologically active agents are cytokines, nerve growth factor, hyaluronic acid, tacrolimus, cyclosporin A, melatonin, vitamin B12, methylprednisolone, riluzole, taxol, cetuximab, 4-aminopyridine (4-AP), Tesamorelin; preferred example is tacrolimus. Biologically active agents may be surface bound and/or be entrapped in a structure defining the medical tissue wrap, e.g. the wall thereof.

Preferably, the wall is formed of a polymer-based material, preferably an elastomer. This has the advantage that a plurality of manufacturing methods may be applied and/or particular characteristics of the material may be provided based on e.g. a polymer unit. In particular, the polymer-based material may be a biocompatible material, which furthermore has elastic properties, such that, in the implanted state, the medical tissue wrap may adapt to tissue movement surrounding the internal anatomical structure to be treated.

The wall may be formed of a polymerized and/or crosslinked polymer unit, which preferably comprises an ester group component and an acid ester group component. The ester group component is preferably a polyol. The acid ester group component is preferably a polyacid.

The material being used for the wall may preferably be the same along its entire circumference and longitudinal extension. Thereby, manufacturing may be further facilitated and structural characteristics of the wall may be essentially homogeneous along the longitudinal direction of the medical tissue wrap. In this matter, if the medical tissue wrap is configured accordingly, biodegradation (and/or bioresorption) may also occur in a predefined or expected manner. However, it may also be provided that at least a portion of the wall is formed of a material being different from the one or more other portions of the wall. Such configuration may be advantageous e.g. if one longitudinal end portion has a particular functionality and may require different characteristics, e.g., if such a longitudinal end portion is configured as a primary inlet for the application of a medical adhesive.

The medical tissue wrap may be formed by a 3D-printing process. This is particularly advantageous when the material of the medical tissue wrap is polymer-based, wherein a curing of the material may be provided essentially instantaneously, for example using (UV) light. Furthermore, this provides an accuracy level that may not be (easily) achieved by means of extrusion and/or a dipping process and/or molding. In particular, the 3-D printing process enables to obtain a particular shape of the medical tissue wrap, wherein, for example, the printing process may provide that particular biologically active agents are integrated in the 3-D structure according to a predefined pattern, e.g. within a mesh structure and/or in particular pockets or cavities formed by the 3-D structure. Thereby, orchestration and support of the tissue repair may be further improved and/or biodegradation may be achieved in a more controllable matter. Moreover, such 3-D printing process may provide the inclusion of local material stresses, which may provide a predefined biasing force so as to facilitate a recoiling function of the wall, e.g. by causing a predefined resilience.

The above object is furthermore achieved by use of a medical tissue wrap described in the above for repairing, supporting, and/or guiding neural tissue, in particular for repairing a peripheral nerve lesion. Furthermore, the nerve wrap may be used in combination with a medical adhesive. Medical adhesive according to the invention may be any medical adhesive of the art. In some embodiments, the medical adhesive is able to polymerize when exposed to light. Before such polymerization the medical adhesive may be fluid or viscous. Preferably, the medical adhesive is a light-curable compound. In some embodiments, said photoinitiator is sensitive to ultraviolet (UV) radiations. According to preferred embodiment the medical adhesive is or comprises poly glycerol sebacate acrylate (PGSA) or PGSAA (e.g., as described in WO 2021/078962).

According to a further aspect, the present invention also provides a kit of part comprising at least one a medical tissue wrap as described above. In some embodiments, the kit of parts comprises at least two (e.g., 1, 2, 3, 4, 5, 6, 7, 8, 9, 10 or more) distinct medical tissue wraps as described above. The distinct medical tissue wraps may differ, e.g., in their dimensions (such as inner diameter and/or longitudinal extension). In some embodiments, the kit of part comprising at least one a medical tissue wrap as described above and a medical adhesive as described above.

According to another aspect of the invention, a method for treating a peripheral nerve lesion is suggested, comprising the steps of:
- providing a medical tissue wrap as described in the above;
- uncoiling the wall of the medical tissue wrap so as to provide a circumferential gap between a circumferential end face of the circumferential inner end portion and a circumferential end face of the circumferential outer end portion;
- inserting the lesioned nerve into the medical tissue wrap via said gap; and
- securing the lesioned nerve within the wall by recoiling the wall.

According to a further aspect, the present invention also provides a method of supporting (protecting) a nerve, comprising the steps of:
- providing a medical tissue wrap according to any of the preceding claims;
- uncoiling the wall of the medical tissue wrap so as to provide a circumferential gap between a circumferential end face of the circumferential inner end portion and a circumferential end face of the circumferential outer end portion;
- inserting the nerve to be supported into the medical tissue wrap via said gap; and
- securing the nerve to be supported within the wall by recoiling the wall.

Preferably, the securing of the nerve (lesioned or to be supported) is furthermore performed by applying a medical adhesive outside of the through-hole, at a circumferential end face of the circumferential outer portion, and/or at opposing longitudinal end portions of the wall and/or at the interface with the nerve tissue.

Similarly, the method of the invention can be used for securing tendon or vessel (lesioned or to be supported).

### Brief description of the drawings

The present disclosure will be more readily appreciated by reference to the following detailed description when being considered in connection with the accompanying drawings in which:
Figure 1 shows a schematic depiction of a medical tissue wrap according to the invention in a schematic perspective view;
Figure 2 shows a schematic depiction of a medical tissue wrap according to the invention in a cross-sectional view;
Figure 3 shows a schematic depiction of a medical tissue wrap according to the invention in a perspective side view and having longitudinal end portions with retention surfaces;
Figure 4 shows the medical tissue wrap according to Figure 3 in a longitudinal sectional view;
Figure 5 shows the medical tissue wrap according to Figure 3 in a cross-sectional view at one longitudinal end portion;
Figures 6 to 9 show schematic depictions of a medical tissue wrap according to the invention in a perspective side view and having end portions with alternative retention surfaces;
Figure 10 shows a schematic depiction of an undercut defined by the groove according to Figure 3; and
Figure 11 shows a cross-sectional view of a medical tissue wrap according to the invention having an alternative configuration of the circumferential inner and outer end portions.
Figure 12 shows a schematic depiction of a medical tissue wrap according to the invention having the outermost edge of the respective longitudinal end portion being offset in a longitudinal direction of the wall.

### Detailed description of preferred embodiments

In the following, the invention will be explained in more detail with reference to the accompanying figures. In the Figures, like elements are denoted by identical reference numerals and repeated description thereof may be omitted in order to avoid redundancies.

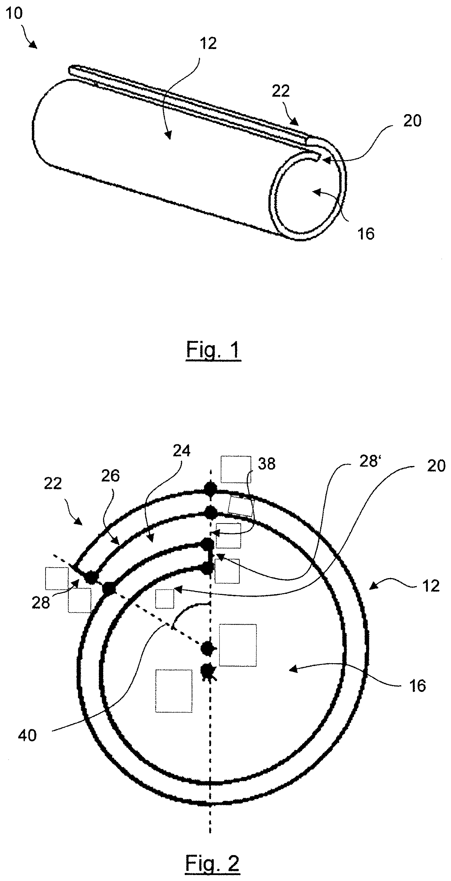

In Figure 1 a medical tissue wrap 10 according to the invention is schematically shown in a perspective view. The medical tissue wrap is essentially formed by a wall 12, which extends in a longitudinal direction and which is coiled so as to define an essentially continuous through-hole 16. The wall 12 is coiled around two longitudinal axes such that, in a coiled state of the wall 12, the through-hole 16 is closed towards the exterior and fully surrounded by the wall along its entire circumference. The closure is provided by a circumferential inner end portion 20 of the wall 12 and a circumferential outer end portion 22 of the wall 12 overlapping the circumferential inner end portion 20, as shown in further detail in Figure 2.

In the present, non-limiting example, the through-hole 16 comprises a tubular shape, in particular having an essentially circular shape in a cross-sectional view, with a continuous inner diameter. Such configuration may be advantageous as it corresponds to the typically cylindrical and/or tube-like extension of the preferred internal anatomical structure to be accommodated within the through-hole 16, in particular a lesioned peripheral nerve. Furthermore, this provides that a predefined and/or more homogeneous structural stability of the medical tissue wrap may be achieved. By the same token, the wall thickness, in the present example, is essentially continuous throughout the entire longitudinal extension and along the circumference of the medical tissue wrap 10. Said wall thickness may be chosen based on the required structural support and resilience of the wall 12 in view of the internal anatomical structure to be treated.

The tubular shape and continuous wall thickness of the wall 12 also provides that essentially continuous outer dimensions may be provided, which may be advantageous for implanting the medical tissue wrap 10 with regard to the surrounding tissue. Furthermore, the tubular or cylindrical shape may provide sufficient structural stability and may prevent sharp bends or kinking during tissue movement, i.e. compression or extension.

As shown in Figure 2, the closure of the through-hole 16 is provided by an overlapping portion of the wall 12 in a circumferential direction. In the coiled state, such overlapping portion is provided by a circumferential outer end portion 22 surrounding a corresponding circumferential inner end portion 20 at the region of the circumferential end faces 28, 28' of the wall 12. In other words, the circumferential extension 40 of the circumferential outer end portion 22 corresponds to the circumferential extension of the circumferential inner end portion 20, such that a full overlap is provided. The circumferential extension 40 indicated in the present example corresponds to about 30 degrees of the outer circumference of the medical tissue wrap 10 as defined by the wall 12. The circumferential extension 40 ensures that no circumferential gap is provided between the circumferential end faces 28, 28' of the wall 12 in the coiled state, such that the internal anatomical structure accommodated within the through-hole 16 is securely retained within said through-hole 16.

Also shown in Figure 2 is that the circumferential outer end portion 22 is spaced apart from the circumferential inner end portion 20 by a radial spacing 24. That is, the circumferential outer end portion 22 is radially spaced apart from the circumferential inner end portion 20 along its entire circumferential extension 40. Thereby, handling of the medical tissue wrap 10 is improved by facilitating an uncoiling of the wall 12 in order to apply the medical tissue wrap 10 around the target tissue to be treated.

The radial spacing 24, however, is discontinuous, meaning that a portion having a reduced radial spacing 24 (but not providing a contacting surface between the circumferential inner and outer end portions 20, 22) is present in the overlapping portion. In the present example, such reduced radial spacing 24 is provided by means of a curvature 26 of the circumferential outer end portion 22, which defines a gradual reduction of the radial spacing 24 from the level of the circumferential end face 28' of the circumferential inner end portion 20 towards the circumferential end face 28 of the circumferential outer end portion 22. Thereby, an improved closure of the through-hole 16 is provided towards the surrounding tissue while maintaining improved usability and uncoiling potential of the medical tissue wrap 10. In particular, the reduction of the radial spacing 24 results in a maximum radial spacing 38 at the level of the circumferential end face 28' of the circumferential inner end portion 20.

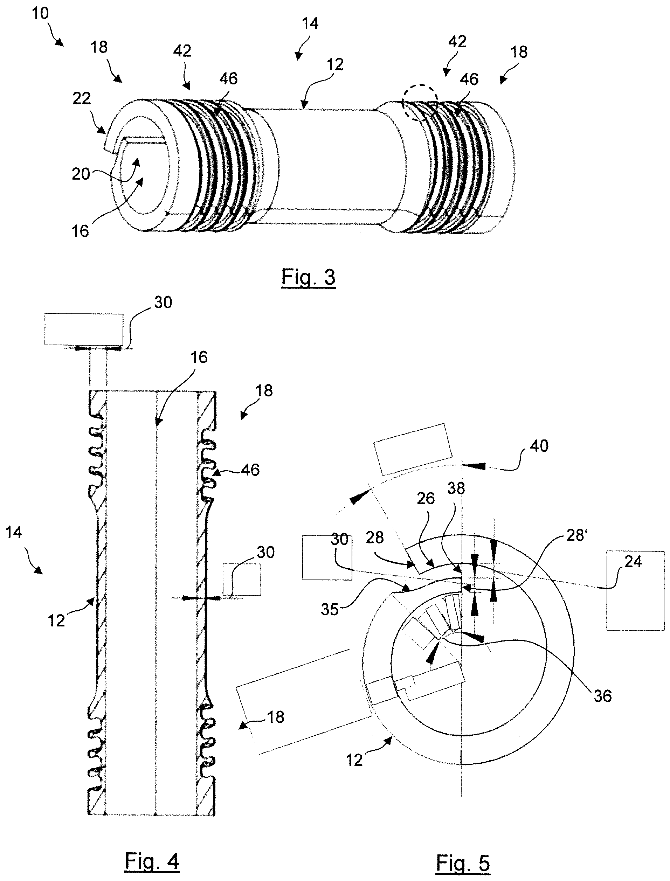

Figures 3 to 5 show different views of an embodiment of a medical tissue wrap 10 having longitudinal end portions 18 with retention surfaces 42 according to the invention. Accordingly, the medical tissue wrap 10 is indicated in a perspective view in Figure 3, wherein the wall 12 defines a central portion 14 and comprises two longitudinal end portions 18, which are arranged at longitudinally opposing ends of the wall 12 and are arranged directly adjacent to the central portion 14. As indicated in the schematic depiction according to Figure 3, the outer surfaces of both longitudinal end portions 18 are aligned or flush with the outer surface of the central portion 14, such that a homogeneous and stepless outer surface is provided that is free of sharp edges, recesses, and/or protrusions potentially adversely affecting the surrounding tissue in the implanted state of the medical tissue wrap 10.

The longitudinal end portions 18 according to the present embodiment are equally shaped and dimensioned, such that during implantation a reversed orientation of the medical tissue wrap 10 does not affect the procedure.

The medical tissue wrap 10 essentially resembles the embodiment according to Figure 1, however, the longitudinal end portions 18 have been provided with retention surfaces 42 configured to facilitate the securing of a medical adhesive to the respective longitudinal end portion 18 at the outer surface of the wall 12. In alternative, optional, embodiments, the retention surfaces 42 may at least partially be also comprised on the central portion 14 being directly adjacent to the respective longitudinal end portion 18.

According to the embodiment depicted in Figures 3 to 5, a plurality of retention surfaces 42 is provided in the form of grooves 46, which extend helically along an outer surface of the wall 12 and along a longitudinal axis defined by said wall 12. The helical shape of the grooves 46 essentially defines a thread shape extending along the outer surface of the respective longitudinal end portion 18. Said grooves 46 or threads are formed in a mirrored arrangement about a transverse mid plane of the wall 12, such that they are aligned in opposing directions, i.e. extending in clockwise and counter-clockwise direction. Although the number of revolutions may vary, the present embodiment depicts the grooves 46 having four revolutions, which has been found to be particularly advantageous in terms of facilitating the securing of the medical adhesive while maintaining structural stability and flexibility of the medical tissue wrap 10. The grooves 46 furthermore end at a longitudinal offset to a longitudinal end surface of the respective end portion 18, which is furthermore advantageous in view of structural stability.

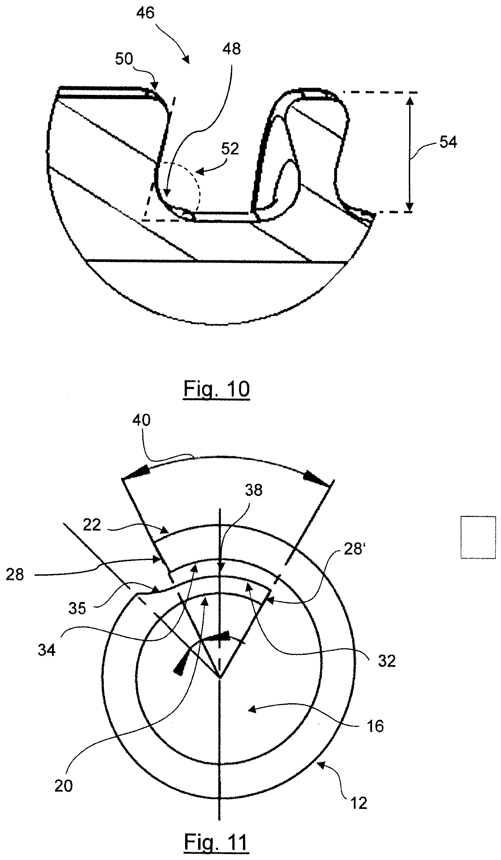

As will be shown in further detail below in view of Figure 10, which is indicated by the dashed circle in Figure 3, the grooves 46 may furthermore comprise rounded edges and define an essentially continuous undercut, enhancing the efficacy of the medical adhesive by forming a form-fitting geometry.

The structural outer surface modifications in the form of retention surfaces 42 are furthermore provided on a thickened portion of the wall 12 compared with the thickness 30 of the wall 12 defining the central portion 14, as shown in a direct comparison in the longitudinal section view of Figure 4.

In Figure 5, the radial spacing 24, is discontinuous, meaning that a portion having a reduced radial spacing 24 is present in the overlapping portion. In particular, the reduction of the radial spacing 24 results in a maximum radial spacing 38 at the level of the circumferential end face 28' of the circumferential inner end portion 20. In the present example, such reduced radial spacing 24 is provided by means of a curvature of the circumferential outer end portion 22. More particularly, the reduced radial spacing 24 at a circumferential end face (28, 28') is about 90 percent of a maximum radial spacing 38 of the discontinuous spacing.

As described above, the longitudinal end portions 18 comprise structural outer surface modifications in the form of retention surfaces 42, which are provided on a thickened portion of the wall 12. In view thereof, the wall 12 of the circumferential inner end portion 20 is thinner, in circumferential direction, as compared to the (thickened) wall 12 of the outer end portion 22 and of (the majority of) the non-overlapping circumferential portion of the (thickened) wall 12. However, as shown in Figure 5, also the non-overlapping circumferential portion of the wall 12 may contain a section 35 having a reduced wall thickness, which is directly adjacent to the circumferential inner end portion 20 having a reduced wall thickness. Thereby, a smooth transition may be provided between (i) the essentially constant wall thickness of the outer end portion and the (majority of the) non-overlapping circumferential portion of the wall 12; and (ii) the inner end portion 20 having a reduced wall thickness in comparison to (i). Accordingly, the thickness of the wall may be gradually reduced (starting) in the section 35 of the non-overlapping circumferential portion of the wall 12 towards the inner end portion 20, reaching a minimum thickness of the wall (in circumferential direction) at the (circumferential end face 28' of the) inner end portion 20. Thereby, sharp edges are preferably avoided, such that a homogeneous and stepless outer surface is preferably provided that is free of sharp edges. Thinning section 35 of the non-overlapping circumferential portion of the wall 12 is provided at a circumferential offset 36. Due to the inner end portion and, optionally, additionally the adjacent section 35 of the non-overlapping circumferential portion of the wall 12, having a reduced wall thickness, manufacturing may be facilitated and the circumferential outer end portion 22 may be better aligned with the adjacent outer circumference of the wall. Furthermore, this may provide that the handling of the medical tissue wrap 10, in particular the accessibility of the circumferential outer end portion 22 is facilitated, such that an uncoiling of the wall 12 and the medical tissue wrap 10 may be supported, both by providing a visual cue and by providing a small gripping recess.

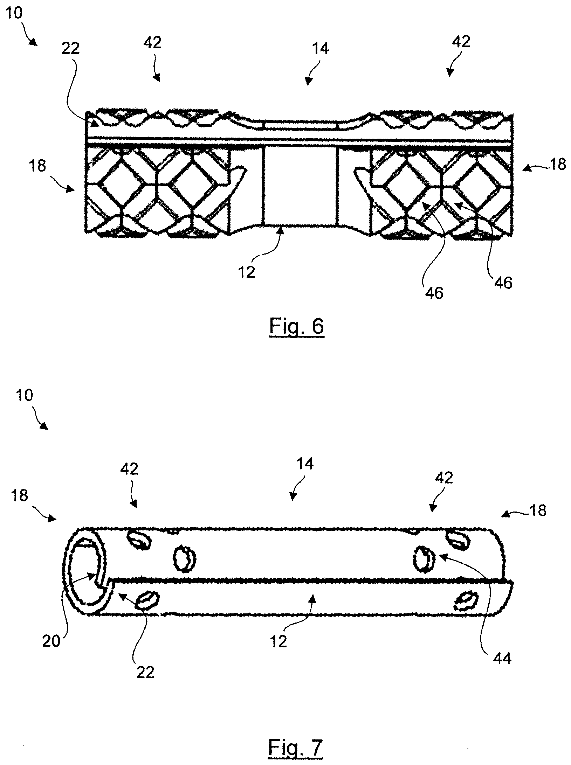

Figures 6 to 9 depict alternative embodiments of the retention surfaces 42 of the embodiment according to Figures 3 to 5. Accordingly, Figure 6 depicts an embodiment of retention surfaces 42 at the opposing longitudinal end portions 18, wherein each longitudinal end portion 18 comprises a plurality of grooves 46 extending in opposing circumferential directions and intersecting each other, as indicated by the corresponding arrows. According to the embodiment, four grooves 46 are provided in each circumferential direction at each respective end portion 18 and the number of grooves 46 is equal for each circumferential direction. As shown in Figure 6, the plurality of grooves 46 in each circumferential direction together with their half-revolution along a longitudinal axis defined by the wall 12 in a helical manner provides a diamond-shape or rhombic-shape at the outer surface of the respective longitudinal end portion 18, which tends to resemble a pineapple surface. Thereby, a plurality of retention surfaces 42 with a large number of edges may be provided, which may be advantageous for securing the medical adhesive to the respective end portion 18. Such pineapple surface may also be provided by providing a plurality of rhombic chambers, wherein the grooves 46 of opposing circumferential directions are not fully in-line with each other.

In the embodiment according to Figure 7, the plurality of retention surfaces 42 are provided in the form of holes 44. The holes 44 according to this example are essentially circular yet being of slight ellipsoid shape, which may occur e.g. during manufacturing, and are formed as through-holes through the wall 12 defining the medical tissue wrap 10. In the exemplary embodiment, the holes 44 are arranged in two longitudinally spaced-apart rows in a circumferential direction of the wall 12, i.e. in a linear fashion along the circumference of the wall 12 within each row and with a longitudinal offset of the rows to each other. One row of holes 44 of a respective longitudinal end portion 14 may also be (at least partially) provided at the central portion 14 being directly adjacent to the respective longitudinal end portion 18 or at the interface between the central portion 14 and the respective longitudinal end portion 18. In the absence of a varying wall thickness, the central portion 14 and the longitudinal portions 18 may e.g. also be characterized by means of the structural and/or (bio)chemical properties and/or the material used for said wall portion. It will also be understood that the holes 44 may also be exclusively at the respective longitudinal end portion 18. Each row comprises six holes 44 that are equally spaced apart in the circumferential direction of the wall 12 and are arranged in a staggered formation between the adjacent rows.

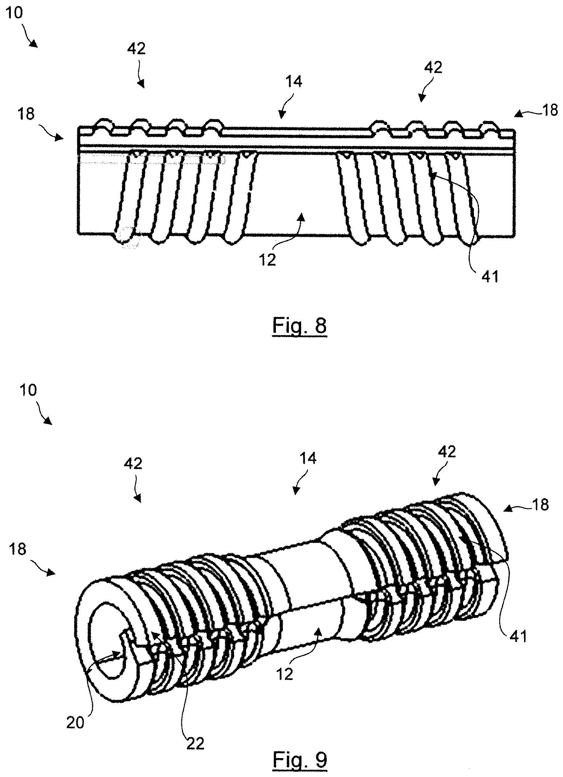

The embodiments according to Figures 8 and 9 essentially resemble the embodiment according to Figure 3. However, instead of providing embedded grooves in a thickened wall portion at the longitudinal end portions, the longitudinal end portions 18 comprise a plurality of positive, e.g. built-up, ribs 41 on top of the otherwise essentially continuous wall defining the through-hole 16 and the medical tissue wrap 10. Said ribs 41 are arranged in an essentially helical fashion according to the embodiment of Figure 8 whereas the ribs 41 according to the embodiment of Figure 9 are arranged in a linear and circumferential manner.

Figure 10 shows a schematic depiction of an undercut defined by the grooves 46 according to the dashed section indicated in Figure 3 in a longitudinal section. As shown, a top outer surface of the groove 46 and a bottom surface of the groove 46, respectively corresponding to an outer radius 50 and an inner radius 48 and defining a groove depth 54, are rounded so as to reduce stress otherwise occurring in the material having grooves 46 with sharp edges. Furthermore, the top surface extends over the bottom surface in a longitudinal direction, thereby forming an angle 52 and corresponding undercut of the groove 46 at the bottom surface, as indicated by the dashed lines, wherein the bottom longitudinal extension may be larger than a top longitudinal extension of the groove 46. The angle 52, preferably about 75 degrees, and corresponding undercut enable that a form-fitting or positive locking between an applied medical adhesive. The respective longitudinal end portion 18 may be provided also in the radial direction, further improving the efficacy of the medical adhesive to secure the internal anatomical structure at the respective longitudinal end portion 18.

Figure 11 shows a cross-sectional view of a medical tissue wrap 10 according to the invention having an alternative configuration of the circumferential inner and outer end portions 20, 22. The embodiment may essentially resemble the embodiment depicted in Figure 5, however, in the present example, the circumferential inner end portion 20 and the circumferential outer end portion 22 each comprise a curvature, wherein said curvatures extend in opposing circumferential directions. Thereby, a gradually reduced radial spacing 24 is provided towards each of the circumferential end faces 28, 28', wherein the radial spacing 24 is reduced starting from a local maximum radial spacing 38.

The maximum radial spacing 38 depicted in this example is arranged such that the circumferential extension 40 of each curvature is essentially the same, i.e. each curvature spans about an equal angle of the outer circumference of the medical tissue wrap 10. For example, the total angle or circumferential extension of the circumferential outer end portion 22 may be about 60 degrees of the outer circumference of the medical tissue wrap 10, wherein each curvature hence spans an angle of about 30 degrees.

Similarly as the embodiment shown in Figure 5, also the embodiment of Figure 11 comprises an inner end portion 20 having a reduced wall thickness and a section 35 of the non-overlapping portion of the wall 12, which has a reduced wall thickness, and which is located adjacent to the inner end portion 20. As shown in Figure 11, in circumferential direction, the wall 12 of the circumferential inner end portion 20 is thinner as compared to the (thickened) wall 12 of the outer end portion 22 and of (the majority of) the non-overlapping circumferential portion of the (thickened) wall 12.

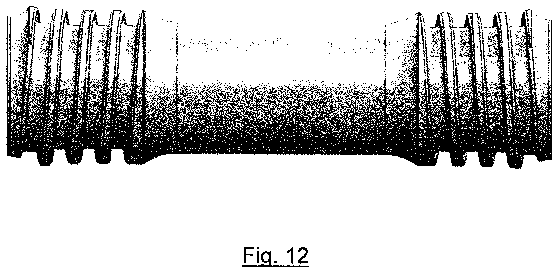

Figure 12 shows a schematic depiction of a medical tissue wrap according to the invention having the outermost edge of the respective longitudinal end portion being offset in a longitudinal direction of the wall, the groove starts so that the additional thickness at the conduit edge is minimized.

It will be obvious for a person skilled in the art that these embodiments and items only depict examples of a plurality of possibilities. Hence, the embodiments shown here should not be understood to form a limitation of these features and configurations. Any possible combination and configuration of the described features can be chosen according to the scope of the invention.

### List of reference numerals

- 10: Medical tissue wrap
- 12: Wall
- 14: Central portion
- 16: Through-hole
- 18: Longitudinal end portion
- 20: Circumferential inner end portion
- 22: Circumferential outer end portion
- 24: Radial spacing
- 26: Curvature
- 28, 28': Circumferential end face
- 30: Wall thickness
- 32: Inner curvature
- 34: Outer curvature
- 35: Section of the non-overlapping circumferential portion of the wall having a reduced wall thickness
- 36: Offset
- 38: Maximum radial spacing
- 40: Circumferential extension
- 41: Rib
- 42: Retention surface
- 44: Hole
- 46: Groove
- 48: Inner radius
- 50: Outer radius
- 52: Angle
- 54: Groove depth

### Items

1. A medical tissue wrap (10) for an internal anatomical structure of a mammal, the tissue wrap (10) being formed by a coiled wall (12) having a circumferential inner end portion (20) and a circumferential outer end portion (22) extending around and overlapping the circumferential inner end portion (20) thereby defining a longitudinal through-hole (16) for accommodating at least one portion of the internal anatomical structure, wherein the circumferential outer end portion (22) is radially spaced-apart from the circumferential inner end portion (20) by a discontinuous spacing in the circumferential direction and the circumferential outer end portion (22) is not in any direct contact with the circumferential inner end portion (20) .
2. The medical tissue wrap (10) according to item 1, wherein the discontinuous spacing comprises a reduced radial spacing (24) formed by a curvature (26) of the circumferential inner end portion (20) and/or a curvature (26) of the circumferential outer end portion (22).
3. The medical tissue wrap (10) according to item 2, wherein the reduced radial spacing (24) is formed as a gradually reduced spacing; and/or wherein the reduced radial spacing (24) extends from an overlapping portion having a continuous radial spacing.
4. The medical tissue wrap (10) according to any one of the preceding items, wherein the radial spacing (24) at a circumferential end face (28') of the circumferential inner end portion (20) is less than the radial spacing (24) at a circumferential end face (28) of the circumferential outer end portion (22).
5. The medical tissue wrap (10) according to any one of items 1 to 3, wherein the radial spacing (24) at a circumferential end face (28) of the circumferential outer end portion (22) is less than the radial spacing (24) at a circumferential end face (28') of the circumferential inner end portion (20).
6. The medical tissue wrap (10) according to any of items 1 to 3, wherein the radial spacing (24) at a circumferential end face (28') of the circumferential inner end portion (20) and at a circumferential end face (28) of the circumferential outer end portion (22) is less than the radial spacing (24) at an overlapping portion between the circumferential end face (28') of the circumferential inner end portion (20) and the circumferential end face (28) of the circumferential outer end portion (22).
7. The medical tissue wrap (10) according to any of items 4 to 6, wherein the radial spacing (24) at a circumferential end face (28, 28') defining the reduced radial spacing (24) is from 50 percent to 99 percent of a maximum radial spacing (38) of the discontinuous spacing, preferably from 60 percent to 99 percent, more preferably from 80 percent to 98 percent, even more preferably from 85 percent to 95 percent, still more preferably from 87 percent to 92 percent, e.g. about 90 percent.
8. The medical tissue wrap (10) according to any of items 4 to 7, wherein the radial spacing (24) at a circumferential end face (28, 28') defining the reduced radial spacing (24) is from 50 µm to 300 µm, preferably from 80 µm to 200 µm.
9. The medical tissue wrap (10) according to any of the preceding items, wherein a maximum radial spacing (38) of the discontinuous spacing is from 100 µm to 350 µm, preferably from 150 µm to 250 µm.
10. The medical tissue wrap (10) according to any of the preceding items, wherein the circumferential extension (40) of the circumferential outer end portion (22) corresponds to from 10 degrees to 100 degrees of the outer circumference of the tissue wrap (10), preferably from 20 degrees to 90 degrees.
11. The medical tissue wrap (10) according to item 10, wherein the circumferential extension (40) of the circumferential outer end portion (22) corresponds to from 20 degrees to 60 degrees of the outer circumference of the tissue wrap (10), preferably from 30 degrees to 45 degrees.
12. The medical tissue wrap (10) according to item 10 or 11, wherein the radial spacing (24) between the circumferential inner end portion (20) and the circumferential outer end portion (22) is gradually reduced in opposing circumferential directions starting from a maximum radial spacing (38) of the discontinuous spacing; preferably with equal angular extension.
13. A medical tissue wrap (10) for an internal anatomical structure of a mammal, the tissue wrap (10) being formed by a coiled wall (12) having a circumferential inner end portion (20) and a circumferential outer end portion (22) extending around and overlapping the circumferential inner end portion (20) thereby defining a longitudinal through-hole (16) for accommodating a portion of the internal anatomical structure, wherein the circumferential outer end portion (22) is radially spaced-apart from the circumferential inner end portion (20) and wherein the wall (12) comprises a structural outer surface modification at least at one opposing longitudinal end portions (18) of the wall (12).
14. The medical tissue wrap (10) according to item 13, wherein a longitudinally extending central portion (14) of the tissue wrap (10) is arranged between and adjacent to the opposing longitudinal end portions (18) of the tissue wrap (10); preferably wherein only the longitudinal end portions (18) comprise the structural outer surface modification.
15. The medical tissue wrap (10) according to item 14, wherein the longitudinal end portions (18) comprise an increased surface roughness as compared with the surface roughness of the outer surface of the central portion (14), which is formed by the structural outer surface modification of the longitudinal end portions (18).
16. The medical tissue wrap (10) according to item 14 or 15, wherein the structural outer surface modifications are formed on a thickened wall portion of the respective longitudinal end portion (18) compared with the wall thickness (30) of the central portion (14).
17. The medical tissue wrap (10) according to item 16, wherein the thickness (30) of the thickened wall portion is from 1,1 to 5,0 fold the thickness (30) of the adjacent central portion (14), preferably from 1,5 to 2,5 fold, and/or is from 100 µm to 600 µm, preferably from 300 µm to 500 µm.
18. The medical tissue wrap (10) according to item 16 or 17, wherein the thickened wall portion is circumferentially offset (36) from the circumferential inner end portion (20), the offset (36) preferably comprising a gradual increase of the wall thickness (30) and/or having a circumferential extension from 5 degrees to 25 degrees of the outer circumference of the tissue wrap (10).
19. The medical tissue wrap (10) according to any of items 13 to 18, wherein the structural outer surface modifications are formed as one or more retention surfaces (42) being configured for securing a medical adhesive to the respective longitudinal end portion (18).
20. The medical tissue wrap (10) according to item 19, wherein the one or more retention surfaces (42) of a respective longitudinal end portion (18) are formed as a plurality of ellipsoid or circular holes (44) arranged in at least one row in a circumferential direction of the wall (12).
21. The medical tissue wrap (10) according to item 20, wherein the holes (44) are arranged in 2 to 4 rows, wherein each row preferably comprises 4 to 8 holes (44) and/or wherein the holes (44) of adjacent rows are preferably arranged in a staggered formation.
22. The medical tissue wrap (10) according to item 20 or 21, wherein each hole (44) is formed as a cut-out, a recess, or a through-hole in the wall (12).
23. The medical tissue wrap (10) according to item 19, wherein the one or more retention surfaces (42) of a respective longitudinal end portion (18) are formed as at least one groove (46) extending in a helical direction along a longitudinal axis defined by the wall (12).
24. The medical tissue wrap (10) according to item 23, wherein the at least one groove (46) comprises rounded edges and/or wherein the at least one groove (46) defines at least one undercut.
25. The medical tissue wrap (10) according to item 23 or 24, wherein the at least one groove (46) defines an outermost edge of the respective longitudinal end portion (18) in a longitudinal direction of the wall (12).
26. The medical tissue wrap (10) according to any of items 23 to 25, wherein the at least one groove (46) extends from 0,5 to 10 revolutions around a longitudinal axis defined by the wall (12).
27. The medical tissue wrap (10) according to item 26, wherein the at least one groove (46) extends from 2 to 6 revolutions around the longitudinal axis defined by the wall (12).
28. The medical tissue wrap (10) according to item 26, wherein the respective longitudinal end portion (18) comprises at least two grooves (46) extending in opposing circumferential directions and intersecting each other, each groove (46) extending from 0,5 to 5 revolutions around a longitudinal axis.
29. The medical tissue wrap (10) according to item 19, wherein the one or more retention surfaces (42) of a respective longitudinal end portion (18) are formed as one or more circumferential ribs (41) extending from an outer surface of the wall (12).
30. The medical tissue wrap (10) according to item 29, wherein the extension of the one or more ribs (41) is linear to the circumferential direction or comprises an offset to the circumferential direction in a longitudinal direction of the wall (12).
31. The medical tissue wrap (10) according to any of items 1 to 12, wherein the wall (12) comprises a structural outer surface modification at least at opposing longitudinal end portions (18) of the wall (12) as defined in any one of items 13 to 30.
32. The medical tissue wrap (10) according to any of the preceding items, the medical tissue wrap (10) being configured to accommodate neural tissue, preferably a lesioned peripheral nerve, more preferably a proximal end and distal end of a lesioned single peripheral nerve.
33. The medical tissue wrap (10) according to any of the preceding items, wherein circumferential outer end portion (22) is formed by a single wall layer overlapping the circumferential inner end portion (20).
34. The medical tissue wrap (10) according to any of the preceding items, wherein the wall (12) is coiled only in a circumferential direction
35. The medical tissue wrap (10) according to any of the preceding items, wherein the circumferential inner end portion (20) and the circumferential outer end portion (22) are arranged at circumferentially opposing end portions (20, 22) of the wall (12).
36. The medical tissue wrap (10) according to any of the preceding items, wherein the wall (12) is at least partially uncoilable and recoilable in a circumferential direction.
37. The medical tissue wrap (10) according to any of the preceding items, wherein the wall (12) is at least partially formed of a resilient or elastic material, preferably of a three-dimensionally printable material.
38. The medical tissue wrap (10) according to any of the preceding items, wherein the wall (12) comprises a thickness (30) from 50 µm to 400 µm, preferably from 150 µm to 250 µm.
39. The medical tissue wrap (10) according to any of the preceding items, wherein the wall (12) is formed of a biocompatible material, an inert material, a bioimplantable material, and/or a biodegradable material.
40. The medical tissue wrap (10) according to any of the preceding items, wherein the wall (12) is formed of a polymer-based material, preferably an elastomer.
41. The medical tissue wrap (10) according to any of the preceding items, wherein the wall (12) is formed of a polymerized and/or crosslinked polymer unit, preferably comprising an ester group component and an acid ester group component, wherein the ester group component is preferably a polyol and/or the acid ester group component is preferably a polyacid.
42. The medical tissue wrap (10) according to any of the preceding items formed by a 3D-printing process.
43. Use of a medical tissue wrap (10) according to any of the preceding items for repairing, supporting, and/or guiding neural tissue, in particular for repairing a peripheral nerve lesion.
44. Use of a medical tissue wrap (10) according to item 43 in combination with a medical adhesive.
45. A method of treating a peripheral nerve lesion, comprising the steps of:
   - providing a medical tissue wrap according to any of the preceding items;
   - uncoiling the wall of the medical tissue wrap so as to provide a circumferential gap between a circumferential end face of the circumferential inner end portion and a circumferential end face of the circumferential outer end portion;
   - inserting the lesioned nerve into the medical tissue wrap via said gap; and
   - securing the lesioned nerve within the wall by recoiling the wall.
46. A method of supporting a nerve, comprising the steps of:
   - providing a medical tissue wrap according to any of the preceding items;
   - uncoiling the wall of the medical tissue wrap so as to provide a circumferential gap between a circumferential end face of the circumferential inner end portion and a circumferential end face of the circumferential outer end portion;
   - inserting the nerve to be supported into the medical tissue wrap via said gap; and
   - securing the nerve to be supported within the wall by recoiling the wall.
47. The method according to item 45 or 46, wherein the step of securing of the nerve is furthermore performed by applying a medical adhesive outside of the through-hole, at a circumferential end face of the circumferential outer portion, and/or at opposing longitudinal end portions of the wall.

## Claims

1. A medical tissue wrap (10) for an internal anatomical structure of a mammal, the tissue wrap (10) being formed by a coiled wall (12) having a circumferential inner end portion (20) and a circumferential outer end portion (22) extending around and overlapping the circumferential inner end portion (20) thereby defining a longitudinal through-hole (16) for accommodating at least one portion of the internal anatomical structure, wherein the circumferential outer end portion (22) is radially spaced-apart from the circumferential inner end portion (20) by a discontinuous spacing in the circumferential direction and the circumferential outer end portion (22) is not in any direct contact with the circumferential inner end portion (20).

2. The medical tissue wrap (10) according to claim 1, wherein the discontinuous spacing comprises a reduced radial spacing (24) formed by a curvature (26) of the circumferential inner end portion (20) and/or a curvature (26) of the circumferential outer end portion (22).

3. The medical tissue wrap (10) according to claim 2, wherein the reduced radial spacing (24) is formed as a gradually reduced spacing; and/or wherein the reduced radial spacing (24) extends from an overlapping portion having a continuous radial spacing.

4. The medical tissue wrap (10) according to any one of the preceding claims, wherein the radial spacing (24) at a circumferential end face (28') of the circumferential inner end portion (20) is less than the radial spacing (24) at a circumferential end face (28) of the circumferential outer end portion (22).

5. The medical tissue wrap (10) according to any one of claims 1 to 3, wherein the radial spacing (24) at a circumferential end face (28) of the circumferential outer end portion (22) is less than the radial spacing (24) at a circumferential end face (28') of the circumferential inner end portion (20),
or
wherein the radial spacing (24) at a circumferential end face (28') of the circumferential inner end portion (20) and at a circumferential end face (28) of the circumferential outer end portion (22) is less than the radial spacing (24) at an overlapping portion between the circumferential end face (28') of the circumferential inner end portion (20) and the circumferential end face (28) of the circumferential outer end portion (22).

6. The medical tissue wrap (10) according to any of claims 4 to 6, wherein the radial spacing (24) at a circumferential end face (28, 28') defining the reduced radial spacing (24) is from 50 percent to 99 percent of a maximum radial spacing (38) of the discontinuous spacing, preferably from 60 percent to 99 percent, more preferably from 80 percent to 98 percent, even more preferably from 85 percent to 95 percent, still more preferably from 87 percent to 92 percent, e.g. about 90 percent,
and/or
wherein the radial spacing (24) at a circumferential end face (28, 28') defining the reduced radial spacing (24) is from 50 µm to 300 µm, preferably from 80 µm to 200 µm.

7. The medical tissue wrap (10) according to any of the preceding claims, wherein a maximum radial spacing (38) of the discontinuous spacing is from 100 µm to 350 µm, preferably from 150 µm to 250 µm.

8. The medical tissue wrap (10) according to any of the preceding claims, wherein the circumferential extension (40) of the circumferential outer end portion (22) corresponds to from 10 degrees to 100 degrees of the outer circumference of the tissue wrap (10), preferably from 20 degrees to 90 degrees,
wherein the circumferential extension (40) of the circumferential outer end portion (22) preferably corresponds to from 20 degrees to 60 degrees of the outer circumference of the tissue wrap (10), preferably from 30 degrees to 45 degrees.

9. The medical tissue wrap (10) according to claim 10 or 11, wherein the radial spacing (24) between the circumferential inner end portion (20) and the circumferential outer end portion (22) is gradually reduced in opposing circumferential directions starting from a maximum radial spacing (38) of the discontinuous spacing; preferably with equal angular extension.

10. The medical tissue wrap (10) according to any of the preceding claims, the medical tissue wrap (10) being configured to accommodate neural tissue, preferably a lesioned peripheral nerve, more preferably a proximal end and distal end of a lesioned single peripheral nerve.

11. The medical tissue wrap (10) according to any of the preceding claims, wherein circumferential outer end portion (22) is formed by a single wall layer overlapping the circumferential inner end portion (20).

12. The medical tissue wrap (10) according to any of the preceding claims, wherein the wall (12) is coiled only in a circumferential direction.

13. The medical tissue wrap (10) according to any of the preceding claims, wherein the circumferential inner end portion (20) and the circumferential outer end portion (22) are arranged at circumferentially opposing end portions (20, 22) of the wall (12).

14. The medical tissue wrap (10) according to any of the preceding claims, wherein the wall (12) is at least partially uncoilable and recoilable in a circumferential direction,
and/or
wherein the wall (12) is at least partially formed of a resilient or elastic material, preferably of a three-dimensionally printable material,
and/or
wherein the wall (12) comprises a thickness (30) from 50 µm to 400 µm, preferably from 150 µm to 250 µm.

15. The medical tissue wrap (10) according to any of the preceding claims, wherein the wall (12) is formed of a biocompatible material, an inert material, a bioimplantable material, and/or a biodegradable material,
and/or
wherein the wall (12) is formed of a polymer-based material, preferably an elastomer,
and/or
wherein the wall (12) is formed of a polymerized and/or crosslinked polymer unit, preferably comprising an ester group component and an acid ester group component, wherein the ester group component is preferably a polyol and/or the acid ester group component is preferably a polyacid.

16. The medical tissue wrap (10) according to any of the preceding claims formed by a 3D-printing process.

17. Use of a medical tissue wrap (10) according to any of the preceding claims for repairing, supporting, and/or guiding neural tissue, in particular for repairing a peripheral nerve lesion,
optionally, in combination with a medical adhesive.
